# EUROPEAN PATENT SPECIFICATION

(11) **EP 2 397 181 B1**
(45) Date of publication and mention of the grant of the patent: **15.01.2014**
(21) Application number: 10166528.9
(22) Date of filing: 18.06.2010
(51) Int. Cl.: A61M 25/06, A61M 5/158

(54) **Insertion device having a permanently locking rotating needle cover**
Einführvorrichtung mit einer permanent verschließenden sich drehenden Nadelabdeckung
Dispositif d'insertion doté d'un étui protecteur d'aiguille rotatif verrouillé de manière permanente

(43) Date of publication of application: 21.12.2011
(73) Proprietor: F. Hoffmann-La Roche AG, 4070 Basel (CH); Roche Diagnostics GmbH, 68305 Mannheim (DE)
(72) Inventor: Pearson, Allen, Wisow Huntington PE28 2QB Cambridgeshire (GB); Collins, James, Yaxley Peterbourough PE7 3LW Cambridgeshire (GB); Pryor, Neil, Willingham CB24 5GD Cambridgeshire (GB)
(74) Representative: Rentsch Partner AG

(56) References cited:
- EP-A1- 1 970 091
- WO-A2-2006/015507

## Description

### Field of the Invention

The invention relates to insertion devices for use with soft infusion cannulas, sensors, and the like, particularly to insertion devices for infusion site interfaces used with infusion pump devices.

### State of the art

Devices for the automated release of liquid medicaments are generally used with patients who have a continuous but varying need of a medicine that can be administered by subcutaneous infusion. Specific applications are for example certain pain therapies and the treatment of diabetes. Infusion pump devices are particularly suitable for self-administration of liquid medicaments. In such cases, computer controlled infusion pump devices are used, which can be carried by the patient on the body, and which contain a certain amount of liquid medicament in a medicine reservoir. The medicine reservoir often comprises medicine sufficient for one to several days. The liquid medicament is supplied to the patient's body from the medicine reservoir through an infusion cannula or an injection needle, which is commonly placed in the subcutaneous body tissue.

A common type of infusion pump systems for continuous subcutaneous infusion comprises an infusion pump device fluidly connected to an infusion set, having an infusion site interface and infusion tubing. The infusion pump device is attached to a convenient place on the patient's body or his clothes, and the infusion tubing is fluidly connected to the pump device with a suitable connector. The infusion site interface is placed at a suitable position on the body, often in the abdominal region around and below the navel. Other possible infusion sites include upper leg, upper buttocks, hips, upper arms and lower back. The infusion site interface contains an infusion cannula, which is inserted into the subcutaneous tissue of the patient. The infusion cannula can be realized as a rigid or semi-rigid hollow needle, or as a soft, flexible cannula, for example in the form of a PTFE cannula. Soft cannulas are generally preferred. The infusion cannula can be inserted perpendicular to the body surface, or at an angle. To avoid medical problems such as infections, the infusion site interface has to be regularly replaced, usually after a few days.

Soft infusion cannulas are well known in the prior art, for both intravenous and subcutaneous infusion. A rigid insertion needle arranged inside the soft cannula facilitates penetration of the skin of the patient and insertion of the cannula into the body tissue. Likewise, hollow sensors for measuring an analyte such as for example glucose are known. After the infusion cannula or the sensor has been placed, the insertion needle is removed from the cannula or sensor, while the soft cannula or sensor remains in the body tissue.

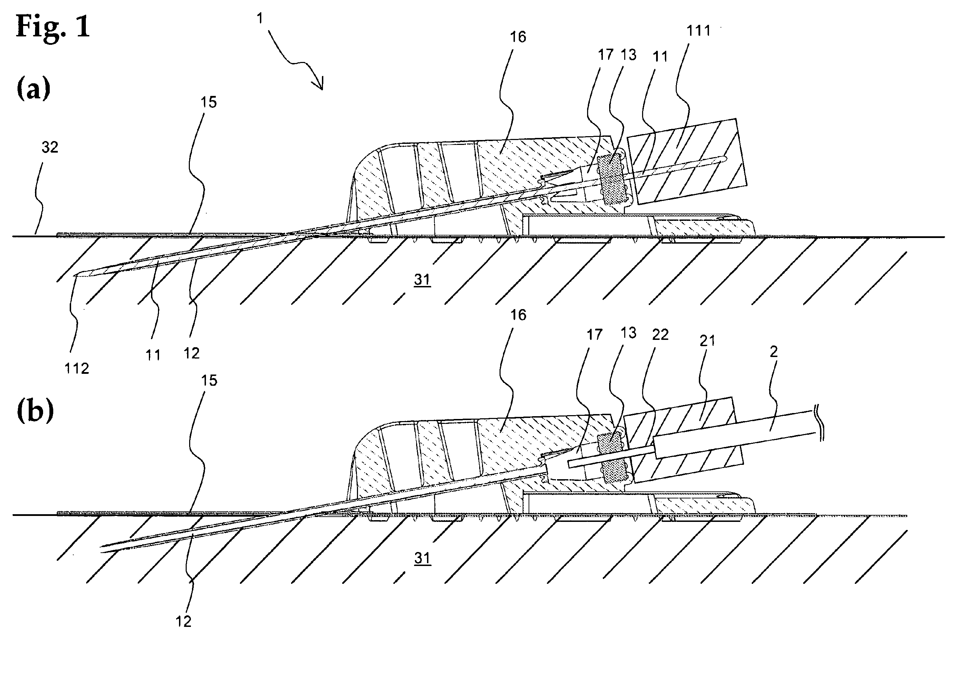

Figure 1 depicts an example of a known infusion site interface 1. A similar infusion site interface as shown in Figure 1 is also known from WO 02/07804 A1. An infusion set with a similar infusion site interface is distributed by Roche Diabetes Care AG, Burgdorf, Switzerland, under the name Accu-Check® TenderLink®. Figure 1(a) shows a cross-section through the infusion site interface 1 attached to the body of a patient, directly after insertion of the infusion cannula. Figure 1(b) shows the infusion site interface 1 after the insertion needle 1 has been removed and the infusion tubing 2 has been connected to the interface 1.

The infusion site interface 1 comprises an interface body 16 mounted on an adhesive pad 15 for attaching the interface to the skin 32 of a patient. An infusion cannula 12 is arranged in an acute angle to the pad 15. One part of the cannula is mounted in the interface body, and the other part projects to the adhesive side of the pad through an opening in the pad. A first end 121 of the cannula 12 opens toward a cavity 17 in the interface body 16, the cavity being sealingly closed by a septum 13. An insertion needle 11 is arranged in the cannula 12, and the septum 13. The needle tip 112 sticks out of the second end 122 of the cannula. On the other end the insertion needle 11 is provided with a handle 111. The handle can be releasably coupled to the interface body 16. If such an interface comprises a sensor instead of an infusion cannula, electronic components of the sensor system can be arranged in the interface body 16..

For mounting the interface 1, the protective cap of the insertion needle (not shown) is removed, cannula 12 and needle 1 are inserted into the subcutaneous tissue 31, the cover foil (not shown) of the adhesive pad 15 is removed, and the pad 15 is adhered to the skin 32 of the patient, finally arriving at Figure 1 (a). The user then removes the insertion needle 11 by grasping the needle handle 111 and retracting the needle 11. The septum 13 sealingly closes the cavity 17. With the needle removed, the infusion cannula 12 establishes a fluid connection between body tissue 31 and cavity 17.

In a next step the infusion tubing 2 of the infusion set is connected to the infusion site interface 1. One end of the infusion tubing is provided with a connector 21 than can be coupled to the interface. The connector 21 comprises a hollow needle 22. When the connector 21 is coupled to the interface 16, the hollow needle 22 penetrates the septum 13 and establishes a fluid connection between the infusion tubing 2 and the cavity 17.

WO 2006/015507 A2 which is considered to define the closest prior art discloses an insertion head for infusion pump devices, in which an insertion needle with handle is removably arranged in an infusion cannula. The infusion cannula is pivotably mounted on a casing of the insertion head. Prior to use, infusion cannula and insertion needle are safely located in the housing of the insertion head. For inserting the cannula into the tissue, the user pivots infusion cannula and insertion needle from the protected position into an insertion position. After insertion of the needle and attaching the insertion head to the patient's skin, the insertion needle is removed by the user and disposed.

Many diabetic patients attach the infusion site interface and insert the infusion cannula or the sensor themselves. However, even with extensive training, the handling with sharp needles unavoidably poses a risk of injuries for patients, as well as medical personnel. In addition, inappropriate disposal of used and thus contaminated insertion needles represents an imminent health risk for third persons, for example, small children. While suitable disposable containers for used needles are generally available in medical facilities, self-care patients in most cases do not have that possibility and have to dispose the insertion needle together with normal domestic waste.

There is a need for devices for inserting cannulas and sensors, and for interfaces having such cannulas and sensors, that allow easy and safe handling by both medical personnel and patients, and that can be safely disposed after use.

### Objects of the Invention

It is an object of this invention to provide a device for inserting a soft cannula or a sensor into the body of a patient that can be safely disposed after use, without the need of special needle disposal containers.

It is another subject of the invention to provide a device for inserting a soft cannula or a sensor into the body of a patient, an infusion site interface together with such a device, and a sensor site interface together with such a device that allows safe handling prior to use and during the insertion of the cannula or sensor.

It is a further subject of the invention to provide such an insertion device that comprises safeguards against incorrect operation by a user.

The device should be producible at low costs. Advantageously it is compatible with existing types of infusion site interfaces.

These and other objects are achieved by a device for inserting a soft cannula or a sensor, an infusion site interface, a sensor site interface, and an infusion set according to the independent claims. Advantageous embodiments are given in the dependent claims.

### Summary of the invention

Within this specification the term user' is meant to signify the person that is applying the device according to the invention, while the term 'patient' is meant to signify the subject to whom the device is applied. Said subject can be a human, but also an animal. In the case of self-administration, the user and the patient are identical.

The present invention can comprise one or more of the features recited in the attached claims, and/or one or more of the following features and combinations thereof

A device according to the invention for inserting an infusion cannula and/or a sensor into a body of a patient comprises an insertion needle adapted to be removably arranged in the infusion cannula and/or sensor, a handle structure connected to the insertion needle, and a needle cover. Said needle cover is pivotably mounted on the handle structure, and is rotatably movable between a first position, where a front part of the insertion needle is located in the needle cover, and a second position, where the front part of the insertion needle is exposed. Advantageously the needle cover comprises a longitudinal slot that is arranged such that the front part of the insertion needle can pass through said slot when the needle cover is pivoted.

An advantageous embodiment of a device according to the invention comprises a lock mechanism for locking the needle cover in the second position.

Another advantageous embodiment of a device according to the invention comprises a spring element adapted to actuate the cover toward the second position.

The spring element in a device according to the invention can comprise an elastic bar that is connected on one end with the needle cover by a first hinge, and is connected on the other end with the handle structure by a second hinge.

Also advantageous is an embodiment of a device according to the invention with a spring element that in a first configuration is non-functional, and in a second configuration is adapted to actuate the needle cover toward the second position. The spring element is adapted to irreversibly change from its first configuration to its second configuration when the needle cover arrives at the second position for the first time. In an advantageous variant of such an embodiment the spring element in its first configuration is adapted to actuate the needle cover toward the first position.

In an even more advantageous embodiment of such a device, one end of the spring element bar comprises a pawl that is adapted to irreversibly snap into a groove in the handle structure when the needle cover arrives at the second position, thereby establishing a second hinge between said end of the bar and the handle structure.

The needle cover of a device according to the invention advantageously comprises an element that is slidably mounted on the handle structure, such that the cover with the sliding element is linearly movable between the first position and a third position.

An especially advantageous variant of the device according to the invention comprises one or more lock mechanisms for locking the needle cover in a position where the needle is located within the needle cover.

In yet another possible variant of such a device, the needle cover comprises an element that is slidably mounted on the handle structure, and is linearly movable between the first position and a third position. A rotational lock mechanism is realized as a slot-tongue joint, wherein a slot is arranged on the cover and the tongue is arranged on the sliding element, or vice versa. The slot and the tongue do not engage when the sliding element is in one position, and do engage when the sliding element is in the other position, thereby blocking the rotational motion of the needle cover and establishing a lock of the needle cover.

In a further advantageous variant of a device according to the invention, the needle cover comprises an element that is slidably mounted on the handle structure, and is linearly movable between the first position and a third position. A rotational lock mechanism comprises a guiding slot. The hinge of the needle cover is located outside the guiding slot when the sliding element is in one position, and is located inside the guiding slot when the sliding element is in the other position, thereby blocking the rotational motion of the needle cover by making the hinge non-functional, and establishing a lock of the needle cover in the first position.

In another possible variant of such a device, the needle cover comprises an element that is slidably mounted on the handle structure, such that the needle cover with the sliding element is linearly movable between the first position and a third position. A translational lock mechanism is realized as a linear ratchet mechanism. The sliding element acts as a linear rack element of the linear ratchet mechanism, and the handle structure comprises a spring loaded pawl of the linear ratchet mechanism, or vice versa. In an especially advantageous embodiment of such a device, the needle cover comprises a closed cap that is arranged such that the tip of the insertion needle is located within the cap when the sliding element is in the third position, thereby positively locking the needle within the cap.

In yet another advantageous variant of a device according to the invention, the needle cover comprises two parallel slots in the two sidewalls of the cover. This provides the possibility that an adhesive pad of an infusion site interface can be arranged within these two slots.

An infusion site interface and a sensor site interface according to the invention comprise an insertion device as discussed above, wherein an insertion needle of the insertion device is arranged in an infusion cannula and/or a sensor of the infusion site interface.

Particularly such an infusion site interface and/or sensor site interface comprises a soft infusion cannula and/or sensor, an insertion needle removably arranged in the infusion cannula and/or sensor, a handle structure connected to the insertion needle, and a needle cover. The needle cover is pivotably mounted on the handle structure, and is rotatably movable between a first position, where a front part of the insertion needle is located in the needle cover, and a second position, where the front part of the insertion needle is exposed.

All advantageous embodiments and variants of insertion devices according to the invention discussed so far are also applicable to an infusion site interface and/or sensor site interface according to the invention.

An infusion set and an infusion pump system according to the invention comprises an insertion device according to the invention, or an infusion site interface according to the invention, respectively.

### Brief description of the drawings

In order to facilitate a fuller understanding of the present invention, reference is now made to the appended drawings. These references should not be construed as limiting the present invention, but are intended to be exemplary only.
- Figure 1: shows a cross-section through a prior art infusion site interface with angled infusion cannula, attached to the body of a patient, (a) directly after insertion of the infusion cannula, and (b) after removal of the insertion needle and with established fluid connection to an infusion tubing.
- Figure 2: shows a cross-section of a cannula insertion device according to the invention, attached to an infusion site interface with a soft cannula, prior to first application. The needle cover is in a first position, with the needle located inside the cover.
- Figure 3: shows a detail view of the device in Figure 2 in the area of the rotational lock mechanism.
- Figure 4: shows a detail view of the device in Figure 2 in the area of the needle tip.
- Figure 5: shows a detail view of the device in Figure 2 in the area of the translational lock mechanism.
- Figure 6: shows a cross-section of the device of Figure 2, with the needle cover in a second, tilted position, in which the needle with surrounding cannula lies free and can be inserted into the body of a patient.
- Figure 7: shows a perspective view of the device of Figure 2, with the needle cover in a second, tilted position, corresponding to Figure 6.
- Figure 8: shows a detail view of the spring element in Figure 6.
- Figure 9: shows the device of Figure 6 after having been removed from the installed infusion site interface, and with the cover pivoted back into the first position.
- Figure 10: shows the device of Figure 9 with the cover irreversibly and permanently locked in a third, final position.
- Figure 11: shows a detail view of the device in Figure 10 in the area of the first rotational lock mechanism.
- Figure 12: shows a second rotational lock mechanism.
- Figure 13: shows a detail view of the device in Figure 10 in the area of the translational lock mechanism.
- Figure 14: schematically shows the different positions of the needle cover in regard to the handle of the device according to the invention.

### Description of embodiments of the invention

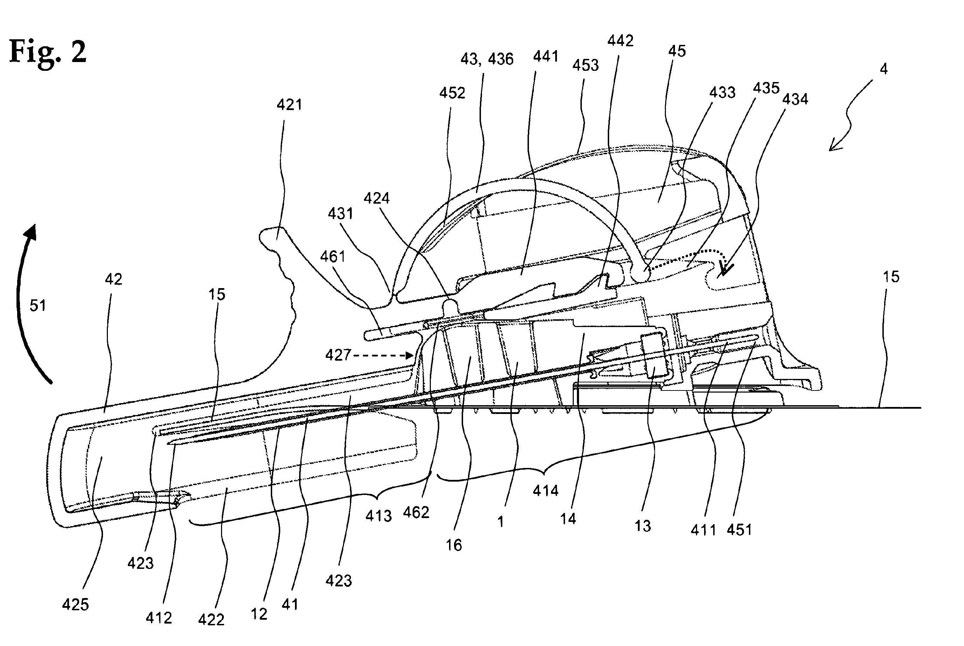

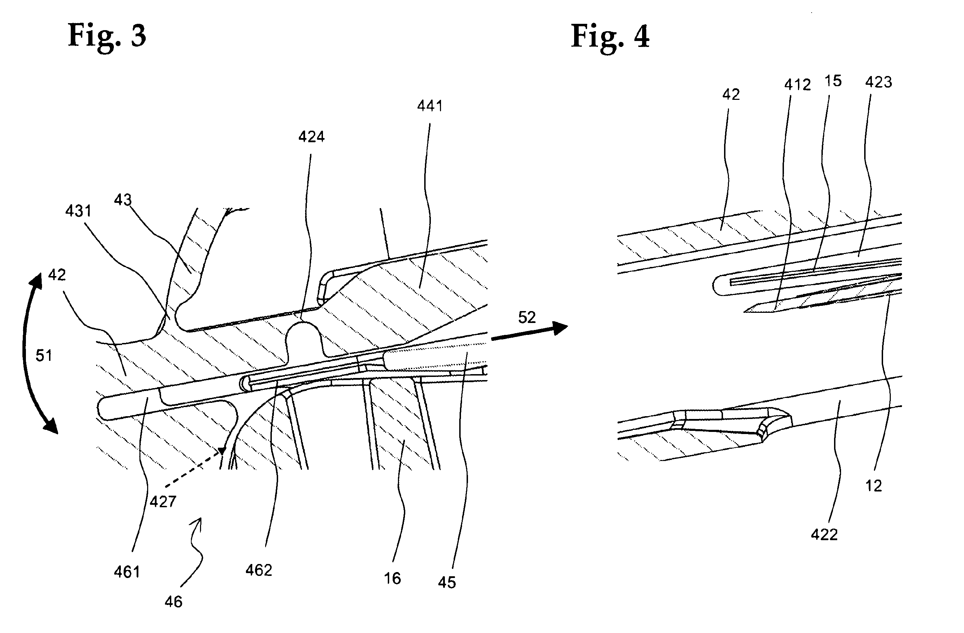

A possible advantageous embodiment of a device 4 for inserting a soft cannula 12 into body tissue is shown in a cross-sectional view in Figure 2. Different detail views of the embodiment are provided in Figures 3, 4, and 5. The disclosed device 4 is particularly suitable for application with an infusion site interface 1 as shown in Figure 1, and is shown already coupled to an infusion site interface 1. The insertion device 4 in combination with the infusion site interface 1 represents an embodiment of an infusion site interface according to the invention.

The infusion site interface shown in Figure 2 is an advantageous infusion site interface with one single septum 12, as well as guiding means that allow a hollow needle of a tubing connector to penetrate the septum 13 parallel to the plane defined by the pad 15 (horizontal in Figure 2), with an angle in regard to the axis of the septum 13 and the cannula 12. However, an insertion device according to the invention can also be used in combination with a prior art infusion site interface, as for example the embodiment shown in Figure 1.

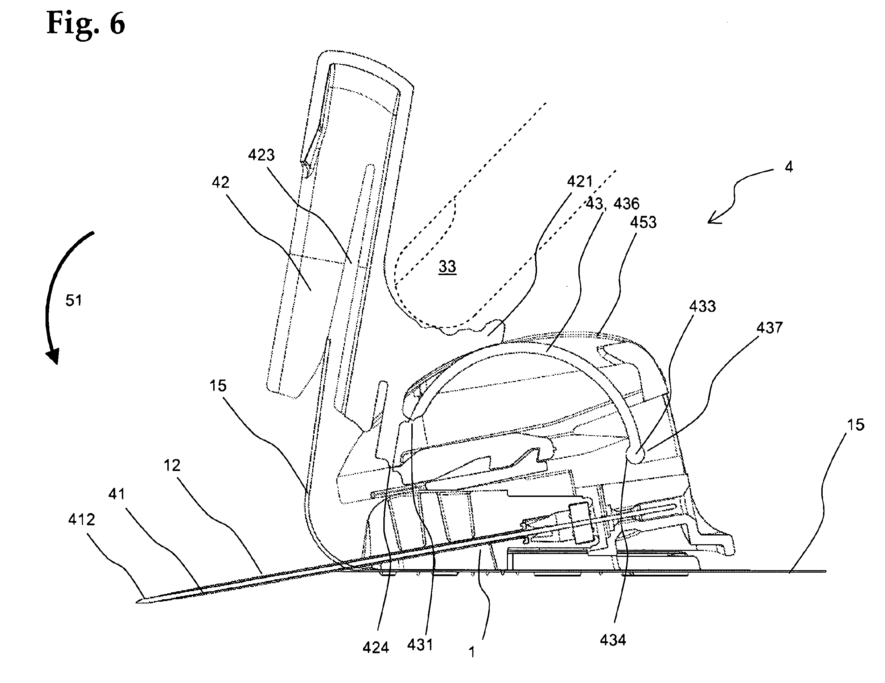

The insertion device 4 is shown in a form as it is provided to a user prior to first use, already assembled with an infusion site interface 1. The device 4 comprises an insertion needle 41 that is arranged in the infusion cannula 12 of the infusion site interface 1, a handle structure 45 that is connected 451 to a first end 41 of the needle, and a needle cover 42 that is pivotably mounted 424 on the handle structure. In the shown embodiment the cover 42 is realized as a sleeve closed at one end, forming a cap 425, and with a longitudinal access slot 422. The cover 42 can be pivoted 51 between a first position and a second position, whereas the insertion needle 41 does not change its position and passes through the access slot 422 of the needle cover 42. The needle cover 42 is provided with a grip 421 that allows a user to actuate the cover by pulling the grip 421 toward the handle frame 45. In the first position, as it is shown in Figure 2, the insertion needle 41 with infusion cannula 12 is located inside the cover 42. The needle tip 412 is not accessible, and there is no risk for a user or patient of getting inadvertently stung. In the second position, as shown in Figure 6, the needle 42 is ready for insertion into the body tissue of a patient.

For being pivotably movable in regard to the frame 45, the cover 42 is connected by hinge 424 to a sliding element 441, slidably mounted in the handle structure 45. In the shown embodiment the hinge is realized as a living hinge. The sliding element 441 is part of a translational lock 44 realized as a linear ratchet mechanism, which will be discussed in more detail further below.

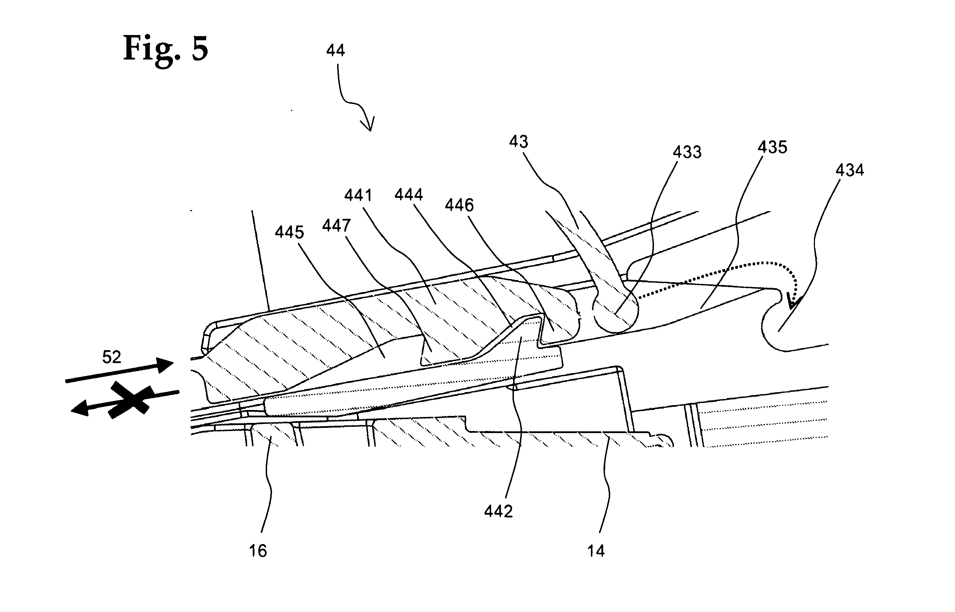

A spring element 43 in the form of an unbiased, curved bar 436 is arranged between the cover 42 and the sliding element 441. It is connected with the cover by a first hinge 431, realized as a living hinge. On the other end of the bar the spring element comprises a pawl 433. When the needle cover 42 is pivoted toward its second position, the first hinge 431 gets bent, and the pawl 433 of the spring element travels on a ramp to the right (dotted arrow in Figures 2 and 5). The spring element 43 is not deformed or biased during this rotational movement 51.

Both the first hinge 431 and the needle cover hinge 424 are advantageously realized as living hinges. Prior to the first rotation movement, the virgin living hinges 431, 424 provide a certain, small resistance that prevents the cover from unwanted rotation during manufacture or handling. The needle tip 412 remains safely located inside the cover 42, and the needle is only accessible when the user actively actuates the rotational motion 51 of the cover 42. After the first rotational actuation, the two hinges 424, 431 do not have such a resistance any more.

In addition to the access slot 422 for the needle 41, the cover 42 furthermore comprises two longitudinal slots 423 on two opposite sides of the cover. The frontal part of the adhesive pad 15 of the infusion site interface 1 is arranged in said two slots 423, as is also visible in Figure 4.

The cover 42, the sliding element 441 and the spring element 43 are advantageously formed as one integral part, and can be manufactured for example by injection molding of a suitable thermoplastic polymer material. The same applies for the handle structure. Prior to assembly the pawl 433 and the sliding element can be connected by a thin predetermined breaking point, which protects the virgin living hinges 431, 424 from being bent during manufacture or assembly, and which is cut during or after assembly of the cover 42 and the handle 45. The insertion needle 41 may be a steel rod or a rigid polymer rod.

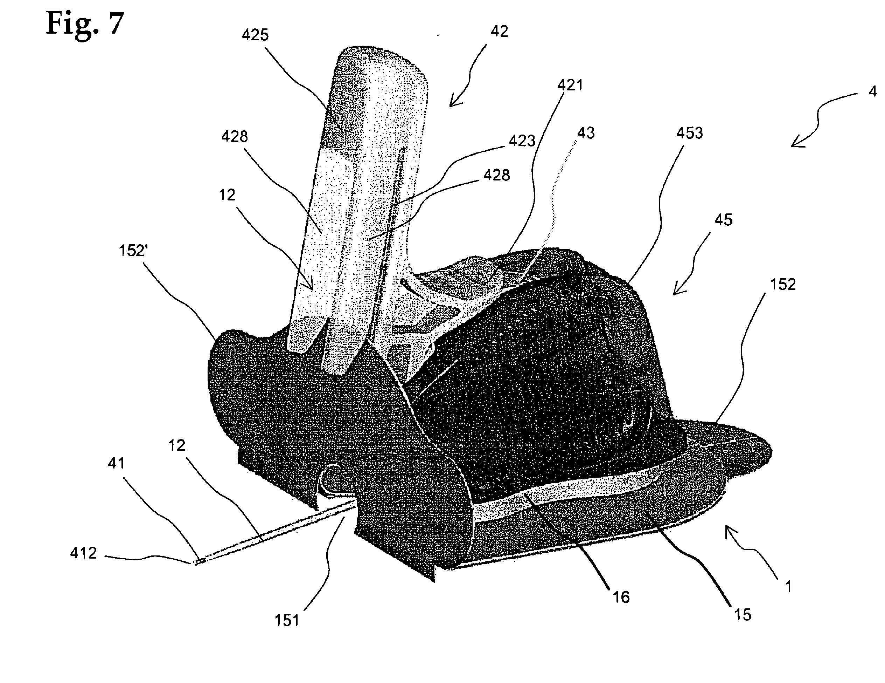

For inserting the needle 41 with the cannula 12 into the body of a patient, the user has to pivot the cover 42 back to a second position, as shown in Figures 6 and 7. For that purpose the user grasps the handle 45 between two fingers (for example thumb and middle finger, not shown) and pulls the grip 421 toward the cover 453 of the handle structure 45 with the index finger 33, until the maximum position is reached ("second position of cover"), where the grip touches the cover 453.

During the pivotal motion, the front end of the adhesive pad 15 slides in the longitudinal slots 423. In the second position of the cover, it is still held by the slots 423, and is bent upwards. The user can easily see the insertion needle, without the need of bending the pad 15 upwards manually. The user can now penetrate the skin 32 of the patient with the now exposed needle 41, and position the needle and cannula 12 within the body tissue.

With the cannula 12 of the interface 1 correctly inserted, the user can then remove the two cover foil parts 152, 152' of the adhesive layer of the pad 15, in order to adhere the pad to the skin 32 of the patient. After this step, the insertion device 4 is removed from the infusion site interface 1, by retracting the insertion needle 41 from the cannula 12 of the interface 1. Alternatively the insertion device can be removed first and the cover foil of the adhesive pad removed later.

In the shown embodiment of the device 4 according to the invention, a mechanism is provided that allows to temporarily lock the needle cover 42 in this second, tilted position, which will now be discussed in more detail. Such a lock has the particular advantage that the user does not have to constantly press the grip 421 of the needle cover, and can more easily remove the device 4 from the interface.

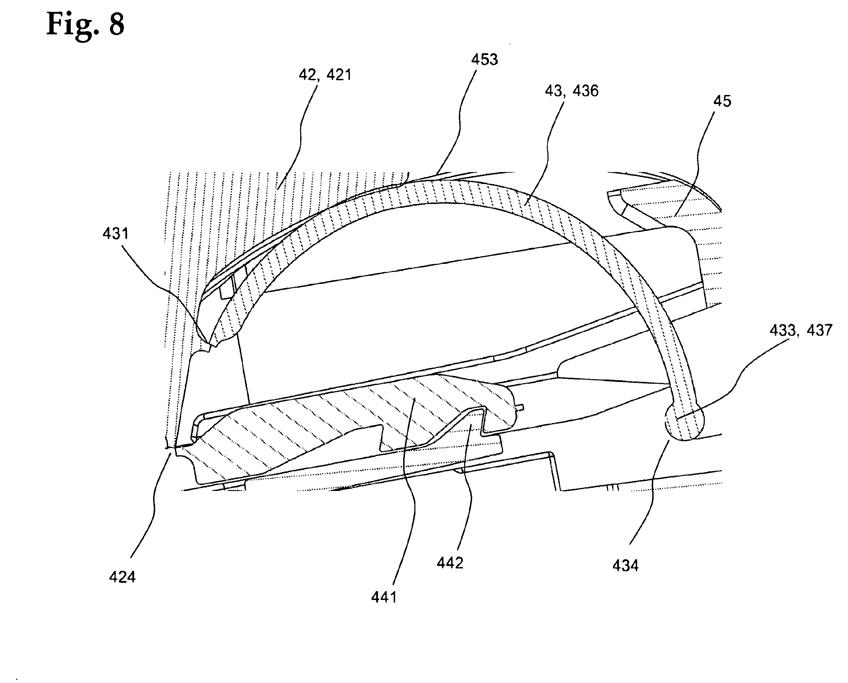

Referring back to Figure 5, one can see the path (dotted arrow) that the pawl 433 of the spring element 43 will take during the first pivotal motion from the first to the second position. The pawl 433 will move to the right, gliding on ramp 435. The pawl 433 is adapted to interact with a corresponding groove 434 provided in the handle structure. When the pawl 433 reaches the end of the ramp 435, it snaps into said groove 434, permanently establishing a second hinge 437 of the spring element 43, as shown in the detail view in Figure 8. The spring element is still unbiased.

For pivoting the cover back towards the first position of Figure 2 the user now has to overcome the spring force of the spring element 43. As a result the user may release the cover grip 421 without having the cover returning to the first position. This is particularly convenient because the user can change the grip position on the handle, or can release the handle completely.

After the user has positioned the cannula 12 within the body of the patient and, if applicable, has attached the interface to the body of the patient, the user will remove the device from the interface by retracting the insertion needle 41 of the device from the cannula 12, the needle cover 42 still being in the tilted, second position.

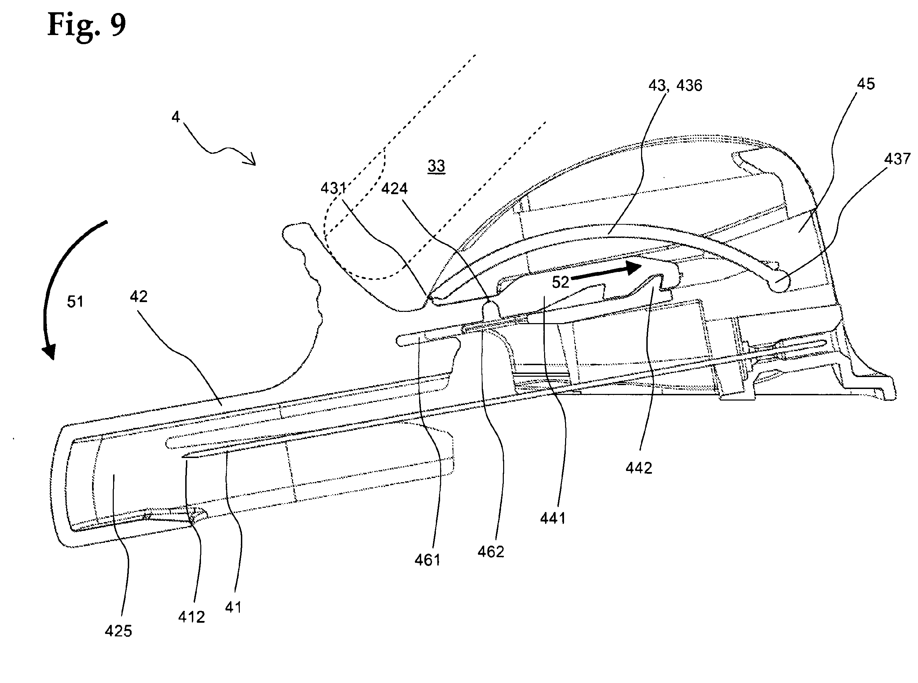

To safely dispose the insertion device 4 with the now exposed insertion needle 41, the user now pivots the needle cover 42 back over the needle 41, arriving back at the first position as shown in Figure 9. However, in contrast to the state of the device in Figure 2, where the spring element 43 in its first configuration is unbiased, the spring element 43 in its second configuration is now elastically deformed and biased and exerts a force.

In order to explain the next steps, the translational lock 44 and the rotational lock 46 are now explained in more detail, by referring back to Figure 3, where a detailed view is given of the rotational lock mechanism 46, and to Figure 5, where a detailed view is given of the translational lock 44.

The rotational lock 46 comprises a tongue 462 connected to the handle structure 45, and a slot 461 in the cover 42. As long as the cover is in the first position of Figure 2, the tongue 462 is located outside of the slot 461, and the rotational movement 51 of the cover 42 around the pivotal point 424 is not hindered. In the situation of Figure 9, however, a spring force is directed toward the handle structure (to the right in Figure 9). Therefore, in the moment the cover 42 reaches the first position, the sliding element 41 will shift from the left to the right along translational direction 52, and the tongue 462 will lock to the slot 461, thereby establishing the rotational lock 46. The cover 42 can no longer pivot 51 back to its second position. This becomes clearer in the detailed view provided in Figure 1 1, where the tongue 462 has reached its final position in the lock mechanism 46. The needle cover 42 of the device 4 is now locked in a position where the needle is located within the needle cover. The needle 41 is no longer exposed, and the device according to the invention can now be safely disposed.

An additional advantageous effect of the tongue 462 is the fact that it can interact with the curved area 427 of the side walls of the cover 42 marked by a dashed arrow in Figures 2 and 3. As long as the cover 42 is not yet back in its first position, the tip of the tongue 462 will slide on the area 427, thereby blocking the movement of the sliding element 441 along direction 52. This feature provides that the locking mechanisms 44, 46, 46' are not prone to jamming, since the locking mechanisms 44, 46, 46' become only functional when the cover reaches the first position.

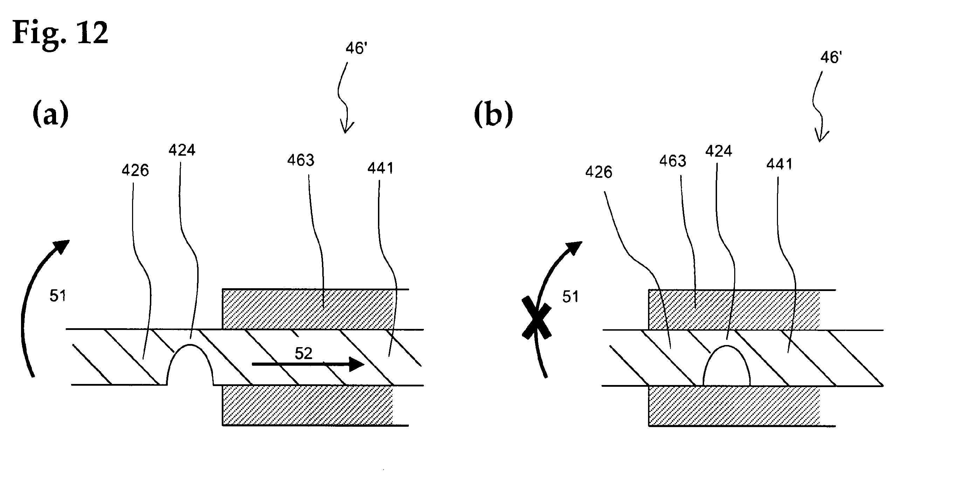

An alternative or additional rotational lock 46' is shown in Figure 12. The portion of the cover close to the hinge 424 is mounted in a guiding slot 463. In the first position (Figure 12(a)), corresponding to Figure 2, the living hinge 424 is located outside of the guiding slot 463 of the rotational lock 46'. The cover 42 can carry out its pivotal motion. Then, driven by the spring force of the spring element, the sliding element 441 is shifted to the right along translational direction 52. The hinge 424 and the adjacent region 426 of the cover 42 are now located in the slot 463. As a result the rotational motion 51 is blocked.

A device according to the invention can be provided with one or both of the rotational lock mechanisms 46, 46' depicted in Figures 1 and 12. An embodiment with both rotational locks is particularly advantageous, since in such a configuration the two portions of the device that are subject to mechanical stress have a certain distance to each other along direction 52. This strongly increases the overall mechanical strength of the rotational lock.

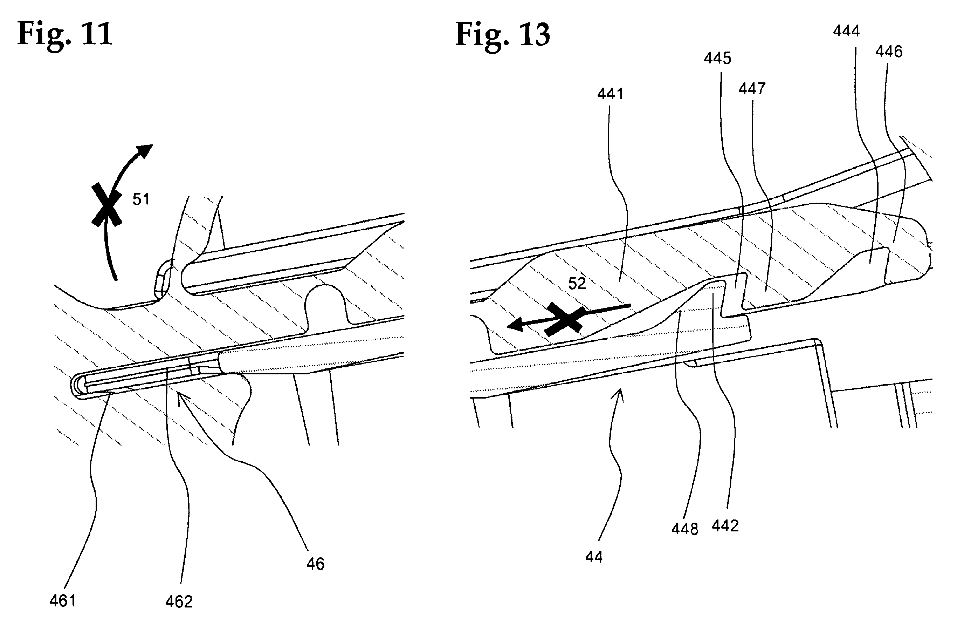

In the shown, particularly advantageous embodiment of the invention, an additional lock mechanism 44 is provided, realized as a linear ratchet mechanism comprising a linear rack and a pawl. As can be seen in Figures 5 and 13 the linear rack is realized as the sliding element 441. A pawl 442 is spring-loadedly mounted on the handle structure 45, interacting with the teeth 446, 447 of the linear rack element 441, which is guided in the guiding slot (not visible). The main purpose of the translational lock 44 is to irreversibly lock the sliding element 441 in a position where the rotational lock(s) 46, 46' are established, thereby permanently locking the cover in its third, final position.

The translational lock 44 can essentially be in two states. In a first state (corresponding to the Figure 5), the pawl 442 is positioned in the first groove 444 of the rack element 441. This is the case in Figures 2 to 9. The sliding element cannot shift to the left, away from the handle structure, since the undercut first tooth 446 of the sliding element hooks into the undercut pawl 442.

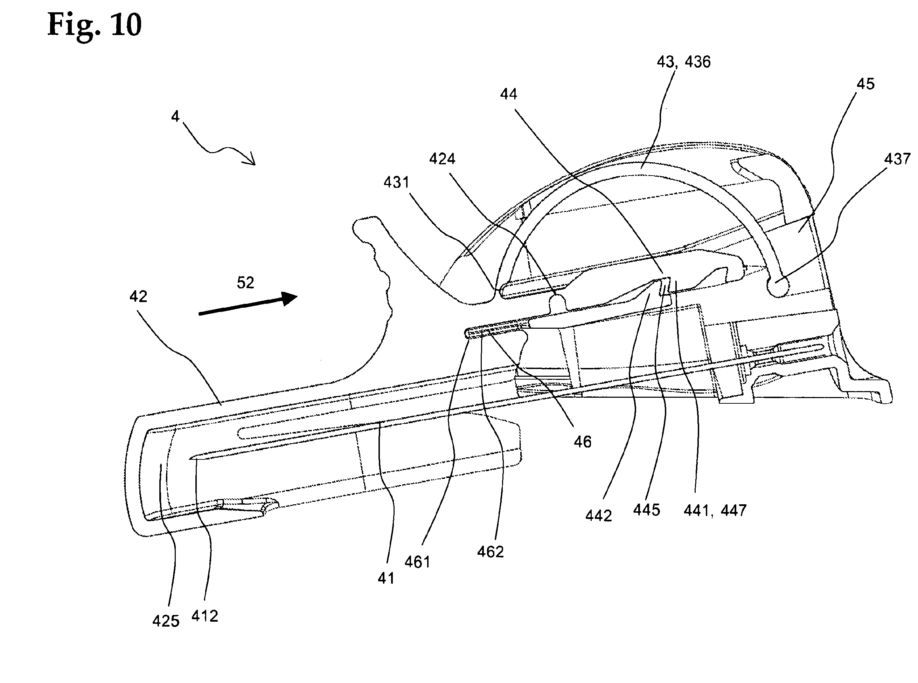

Driven by the tension of the spring element 43 in its new, second configuration (see Figure 9), the sliding element 441 will shift to the right along translational direction 52. The ramp 448 of the pawl 442 glides over the second tooth 447, finally arriving in the situation of Figure 13, where the pawl is now positioned in the second groove 445. The undercut second tooth 447 of the sliding rack element 441 hooks into the undercut pawl 442. The linear ratchet mechanism 44 is permanently locked. The cover of the device 4 is also shifted to the right, and is now in its final, third position, as it is shown in Figure 10. The tip 412 of the insertion needle 41 is inaccessibly located in the closed cap 425 in the front of the cover 42.

In an alternative variant of a device according to the invention, the roles of the sliding rack element and the pawl can be exchanged, the pawl being a part of the sliding element, and the linear rack being part of the handle frame. It is also possible to springload the linear rack instead of the pawl, or even both elements.

It should be mentioned that the shown embodiment of an insertion device according to the invention has the further advantage that it is not possible for the cover 42 to lock into the third position as long as the device is still mounted on an infusion site interface 1. Referring back to Figure 2 and 3, one can see that the shape of the cover in the area 427 marked by a dashed arrow corresponds to the shape of the body 16 of the infusion site interface. As a consequence, if the user erroneously pivots the cover 42 back to its first position before he has inserted the needle 41 and cannula, which is possible, the marked area of the cover will rest on the adjacent interface body 16, thereby rendering the lock mechanisms 44, 46, 46' non-functional, as the cover cannot shift along direction 52. The user cannot accidentally lock the cover prior to use and make the device irreversibly non-functional.

A device according to the invention can also be realized without a rotational lock mechanism 46, 46'. If no rotational lock is provided to the device, the cover nevertheless cannot expose the needle. Since due to the translational lock mechanism 44 the needle tip 412 is shifted into the fully closed cap 425 of the cover 42, the front end of the needle will collide with the cap 425, thereby also preventing the needle from leaving the needle cover.

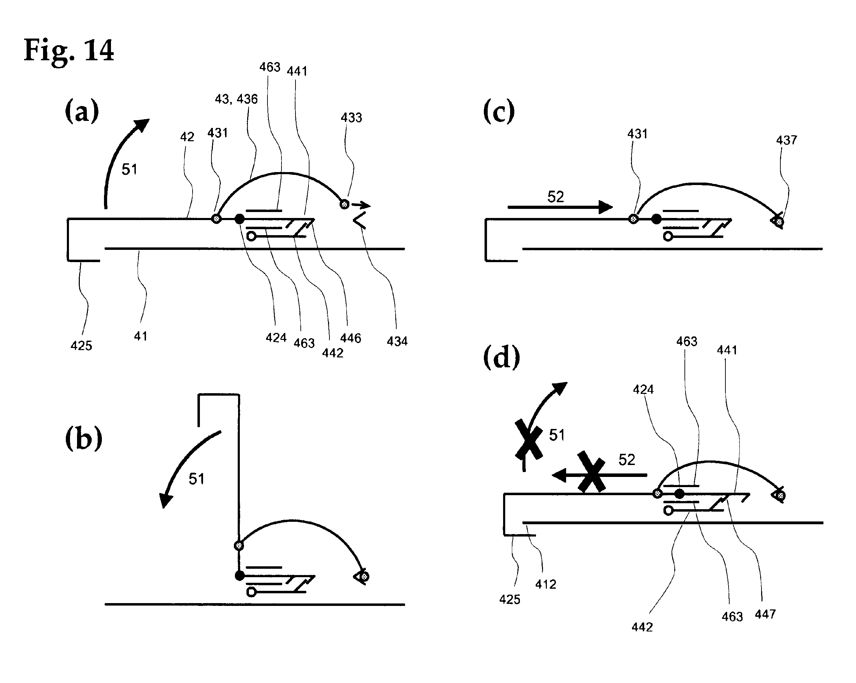

The functionality of the device according to the invention will now be summarized and described in the schemes shown on Figure 14(a) to (d). For a better understanding only the features of the device are shown that are necessary for explaining the basic functionality.

In Figure 14(a) the device 4 is shown with the needle cover 42 in the first position. The spring element 43 is in the first, non-functional configuration. The cover is then pivoted 51 about the cover hinge 424, and is in its second position, as shown in Figure 14(b). The needle 41 is exposed and can be inserted into the body of the patient. The pawl 433 of the spring element 43 snaps into groove 434, thereby establishing the second hinge 437 of the spring element. The spring element is now in its second, functional configuration.

After having removed the device from the interface, the cover is pivoted back to the first position, arriving at Figure 14(c). The spring element generates a force that actuates the cover 42 with sliding element 441 along direction 52. The sliding element/ linear rack 441 is shifted along 52, and the pawl 442 hooks into the second tooth of the linear rack 441. The cover is now in the third position, as shown in Figure 14(d). The cover hinge 424 is located within the guiding slot 463, and is no longer functional. The sliding element is locked by the ratchet mechanism 44. As a result, the needle cover 42 is permanently locked in its rotational position in regard to the handle 45, and the needle tip is located in the cap 425. The device 4 according to the invention can now be safely disposed.

The embodiments of an insertion device and an infusion site interface according to the invention that have been discussed so far are adapted to be used for subcutaneous infusion, particularly in combination with an infusion pump system. However they can also be used for intravenous infusions, with some minor adaptations to the infusion site interface. As already mentioned above, the device according to the invention can also be applied for inserting sensors into a body of a patient, instead of a soft cannula.

### List of Reference Numerals

- 1: infusion site interface
- 11: insertion needle
- 111: handle
- 12: needle tip
- 12: infusion cannula
- 121: first end of infusion cannula
- 122: second end of infusion cannula
- 13: septum
- 15: carrier, adhesive pad
- 151: opening in pad for cannula
- 152, 152': cover foil for adhesive layer
- 16: interface body
- 17: cavity
- 2: infusion tubing
- 21: tubing connector
- 22: hollow needle
- 31: subcutaneous body tissue
- 32: skin
- 33: finger of user
- 4: insertion device
- 41: insertion needle
- 41: first end of the insertion needle
- 412: second end of the insertion needle, needle tip
- 413: front part of the needle
- 414: back part of the needle
- 42: needle cover
- 421: grip
- 422: access slot
- 423: guiding slot for pad
- 424: hinge of needle cover, pivotal point
- 425: cap
- 426: region of cover adjacent to hinge
- 427: curved area of cover sidewalls
- 428: sidewall of cover
- 43: spring element
- 431: first hinge of spring element
- 433: pawl
- 434: groove
- 435: ramp
- 436: curved bar
- 437: second hinge of spring element
- 44: translational lock
- 441: sliding element, linear rack
- 442: pawl
- 444: first groove
- 445: second groove
- 446: first tooth
- 447: second tooth
- 448: ramp
- 45: handle structure
- 451: connection between needle and handle
- 452: slot
- 453: cover
- 46, 46': rotational lock
- 461: slot
- 462: tongue
- 463: guiding slot
- 51: direction of rotational motion
- 52: direction of translational motion

## Claims

1. A device (4) for inserting an infusion cannula (12) and/or a sensor into a body of a patient, comprising an insertion needle (41) adapted to be removably arranged in the infusion cannula (12) and/or sensor, a handle structure (45) connected (451) to the insertion needle, and a needle cover (42), wherein said needle cover (42) is pivotably mounted (424, 441) on the handle structure (45), and is rotatably movable (51) between a first position, where a front part (413) of the insertion needle (41) is located in the needle cover (42), and a second position, where the front part of the insertion needle is exposed, **characterized by** a spring element (43) that in a first configuration is not functional, and in a second configuration is adapted to actuate the needle cover toward the second position, wherein the spring element is adapted to irreversibly change from its first configuration to its second configuration when the needle cover arrives at the second position for the first time.

2. The device according to claim 1, **characterized in that** the needle cover (42) comprises a longitudinal slot (422) that is arranged such that the front part (413) of the insertion needle (41) can pass through said slot when the needle cover is pivoted (51).

3. The device according to claim 1 or 2, **characterized by** a spring element (43) adapted to actuate the cover (42) toward the second position.

4. The device according to any of the preceding claims, **characterized in that** one end of the spring element bar (436) comprises a pawl (433) that is adapted to irreversibly snap into a groove (434) in the handle structure (45) when the needle cover (42) arrives at the second position, thereby establishing a second hinge (437) between said end of the bar (436) and the handle structure (45).

5. The device according to any of the preceding claims, **characterized in that** the spring element (43) comprises an elastic bar (436) that is connected on one end with the needle cover (42) by a first hinge (431), and is connected on the other end with the handle structure (45) by a second hinge (437).

6. The device according to any of the preceding claims, **characterized in that** the needle cover (42) comprises an element (441) that is slidably mounted (52, 463) on the handle structure (45), such that the cover with the sliding element (441) is linearly movable (52) between the first position and a third position.

7. The device according to any of the preceding claims, **characterized by** one or more lock mechanisms (46, 46', 44, 425) for locking the needle cover (42) in a position where the needle (41) is located within the cover (42).

8. The device according to any of the preceding claims, **characterized in that** the needle cover (42) comprises an element (441) that is slidably mounted (52, 463) on the handle structure (45), and is linearly movable (52) between the first position and a third position, and a rotational lock mechanism (46) is realized as a slot-tongue joint, wherein a slot (461) is arranged on the cover (42) and the tongue (462) is arranged on the sliding element (441), or vice versa, and wherein the slot (461) and the tongue (462) do not engage when the sliding element (441) is in one position, and do engage when the sliding element is in the other position, thereby blocking the rotational motion (51) of the needle cover (42) and establishing a lock of the needle cover.

9. The device according to any of the preceding claims, **characterized in that** the needle cover (42) comprises an element (441) that is slidably mounted (52, 463) on the handle structure (45), and is linearly movable (52) between the first position and a third position, and a rotational lock mechanism (46') comprising a guiding slot (463), wherein the hinge (424) of the needle cover is located outside the guiding slot (463) when the sliding element (441) is in one position, and is located inside the guiding slot when the sliding element is in the other position, thereby blocking the rotational motion (51) of the needle cover (42) by making the hinge (424) non-functional, and establishing a lock of the needle cover in the first position.

10. The device according to any of the preceding claims, **characterized in that** the needle cover (42) comprises on element (441) that is slidably (52, 463) mounted on the handle structure (45), such that the needle cover with the sliding element is linearly movable (52) between the first position and a third position, and a translational lock mechanism (44) is realized as a linear ratchet mechanism (441, 442), wherein the sliding element acts as a linear rack element (441) of the linear ratchet mechanism and the handle structure comprises a spring-loaded pawl (442) of the linear ratchet mechanism, or vice versa.

11. The device according to any of the preceding claims, **characterized in that** the needle cover comprises two parallel slots (423) in the sidewalls (428) of the cover (42).

12. An infusion or sensor site interface with an insertion device (4) according to any of claims 1 to 11, wherein an insertion needle (41) of the insertion device (4) is arranged in an infusion cannula (12) or in a sensor of the interface.

13. An infusion set with an insertion device (4) according to any of claims 1 to 11.

## Patentansprüche

1. Vorrichtung (4) zum Einführen einer Infusionskanüle (1 2) und/oder eines Sensors in einen Körper eines Patienten, umfassend eine Einführnadel (41), die so ausgelegt ist, dass sie entfernbar in der Infusionskanüle (12) und/oder im Sensor anordenbar ist, eine Griffstruktur (45), die mit der Einführnadel verbunden (451) ist, und eine Nadelabdeckung (42), wobei die Nadelabdeckung (42) schwenkbar auf der Griffstruktur (45) angebracht (424, 441) und drehbar bewegbar (51) ist zwischen einer ersten Position, in welcher sich ein Vorderteil (413) der Einführnadel (41) in der Nadelabdeckung (42) befindet, und einer zweiten Position, in welcher der Vorderteil der Einführnadel freigelegt ist, **gekennzeichnet durch** ein Federelement (43), das in einer ersten Konfiguration nicht funktionsfähig ist und in einer zweiten Konfiguration so ausgelegt ist, dass es die Nadelabdeckung hin in die zweite Position betätigt, wobei das Federelement so ausgelegt ist, dass es unumkehrbar von seiner ersten Konfiguration in seine zweite Konfiguration wechselt, wenn die Nadelabdeckung zum ersten Mal in der zweiten Position ankommt.

2. Vorrichtung nach Anspruch 1, **dadurch gekennzeichnet, dass** die Nadelabdeckung (42) einen Längsschlitz (422) umfasst, der so angeordnet ist, dass der Vorderteil (413) der Einführnadel (41) den Schlitz passieren kann, wenn die Nadelabdeckung geschwenkt (51) wird.

3. Vorrichtung nach Anspruch 1 oder 2, **gekennzeichnet durch** ein Federelement (43), das so ausgelegt ist, dass es die Abdeckung (42) hin in die zweite Position betätigt.

4. Vorrichtung nach irgendeinem der vorangehenden Ansprüche, **dadurch gekennzeichnet, dass** ein Ende des Federelementstegs (436) eine Klinke (433) umfasst, die so ausgelegt ist, dass sie unumkehrbar in eine Nut (434) in der Griffstruktur (45) einrastet, wenn die Nadelabdeckung (42) in der zweiten Position ankommt, wodurch ein zweites Gelenk (437) zwischen dem genannten Ende des Stegs (436) und der Griffstruktur (45) gebildet wird.

5. Vorrichtung nach irgendeinem der vorangehenden Ansprüche, **dadurch gekennzeichnet, dass** das Federelement (43) einen elastischen Steg (436) umfasst, der an einem Ende über ein erstes Gelenk (431) mit der Nadelabdeckung (42) verbunden ist und am anderen Ende über ein zweites Gelenk (437) mit der Griffstruktur (45) verbunden ist.

6. Vorrichtung nach irgendeinem der vorangehenden Ansprüche, **dadurch gekennzeichnet, dass** die Nadelabdeckung (42) ein Element (441) umfasst, das gleitend an der Griffstruktur (45) angebracht (52, 463) ist, so dass die Abdeckung mit dem Gleitelement (441) linear zwischen der ersten Position und einer dritten Position bewegbar (52) ist.

7. Vorrichtung nach irgendeinem der vorangehenden Ansprüche, **gekennzeichnet durch** einen oder mehrere Verriegelungsmechanismen (46, 46', 44, 425) zum Verriegeln der Nadelabdeckung (42) in einer Position, in der die Nadel (41) innerhalb der Abdeckung (42) angeordnet ist.

8. Vorrichtung nach irgendeinem der vorangehenden Ansprüche, **dadurch gekennzeichnet, dass** die Nadelabdeckung (42) ein Element (441) umfasst, das gleitend an der Griffstruktur (45) angebracht ist (52, 463), und linear zwischen der ersten Position und einer dritten Position bewegbar (52) ist, und ein Drehverriegelungsmechanismus (46) als Nut-Feder-Verbindung umgesetzt ist, wobei eine Nut (461) auf der Abdeckung (42) angeordnet ist und die Feder (462) am Gleitelement (441) angeordnet ist oder umgekehrt, und wobei die Nut (461) und die Feder (462) nicht in Eingriff stehen, wenn sich das Gleitelement (441) in einer Position befindet, und in Eingriff stehen, wenn sich das Gleitelement in der anderen Position befindet, wodurch die Drehbewegung (51) der Nadelabdeckung (42) blockiert und eine Verriegelung der Nadelabdeckung gebildet wird.

9. Vorrichtung nach irgendeinem der vorangehenden Ansprüche, **dadurch gekennzeichnet, dass** die Nadelabdeckung (42) ein Element (441) umfasst, das gleitend an der Griffstruktur (45) angeordnet ist (52, 463), und linear zwischen der ersten Position und einer dritten Position bewegbar (52) ist, und einen Drehverriegelungsmechanismus (46'), der eine Führungsnut (463) umfasst, wobei das Gelenk (424) der Nadelabdeckung ausserhalb der Führungsnut (463) angeordnet ist, wenn sich das Gleitelement (441) in einer Position befindet, und innerhalb der Führungsnut angeordnet ist, wenn sich das Gleitelement in der anderen Position befindet, wodurch die Drehbewegung (51) der Nadelabdeckung (42) blockiert wird, indem das Gelenk (424) nicht-funktionsfähig gemacht wird, und wodurch eine Verriegelung der Nadelabdeckung in der ersten Position gebildet wird.

10. Vorrichtung nach irgendeinem der vorangehenden Ansprüche, **dadurch gekennzeichnet, dass** die Nadelabdeckung (42) ein Element (441) umfasst, das gleitend (52, 463) an der Griffstruktur (45) angebracht ist, so dass die Nadelabdeckung mit dem Gleitelement linear zwischen der ersten Position und einer dritten Position bewegbar (52) ist, und ein translatorischer Verriegelungsmechanismus (44) als linearer Ratschenmechanismus (441, 442) umgesetzt ist, wobei das Gleitelement als lineares Zahnstangenelement (441) des linearen Ratschenmechanismus agiert und die Griffstruktur eine federbelastete Klinke (442) des linearen Ratschenmechanismus umfasst, oder umgekehrt.

11. Vorrichtung nach irgendeinem der vorangehenden Ansprüche, **dadurch gekennzeichnet, dass** die Nadelabdeckung zwei parallele Schlitze (423) in den Seitenwänden (428) der Abdeckung (42) umfasst.

12. Infusions- oder Sensorstellen-Schnittstelle mit einer Einführvorrichtung (4) nach einem der Ansprüche 1 bis 11, wobei eine Einführnadel (41) der Einführvorrichtung (4) in einer Infusionskanüle (12) oder in einem Sensor der Schnittstelle angeordnet ist.

13. Infusionsset mit einer Einführvorrichtung (4) nach einem der Ansprüche 1 bis 11.

## Revendications

1. Dispositif (4) pour insérer une canule de perfusion (12) et/ou un capteur dans un corps d'un patient, comprenant une aiguille d'insertion (41) agencée pour être disposée de façon amovible dans la canule de perfusion (12) et/ou le capteur, une structure de poignée (45) reliée (541) à l'aiguille d'insertion, et un étui protecteur d'aiguille (42), ledit étui protecteur d'aiguille (42) étant monté de façon pivotante (424, 441) sur la structure de poignée (45) et étant déplaçable de façon rotative (51) entre une première position, dans laquelle une partie avant (413) de l'aiguille d'insertion (41) se situe dans l'étui protecteur d'aiguille (42), et une deuxième position, dans laquelle la partie avant de l'aiguille d'insertion est exposée, **caractérisé par** un élément ressort (43) qui, dans une première configuration, n'est pas fonctionnel et, dans une seconde configuration, est apte à actionner l'étui protecteur d'aiguille vers la deuxième position, l'élément ressort étant agencé pour passer de façon irréversible de sa première configuration à sa seconde configuration lorsque l'étui protecteur d'aiguille arrive à la deuxième position pour la première fois.

2. Dispositif selon la revendication 1, **caractérisé par le fait que** l'étui protecteur d'aiguille (42) comprend une fente longitudinale (422) qui est agencée de telle sorte que la partie avant (413) de l'aiguille d'insertion (41) peut passer à travers ladite fente lorsque l'étui protecteur d'aiguille est amené à pivoter (51).

3. Dispositif selon l'une des revendications 1 ou 2, **caractérisé par** un élément ressort (43) agencé pour actionner l'étui protecteur (42) vers la deuxième position.

4. Dispositif selon l'une quelconque des revendications précédentes, **caractérisé par le fait qu'**une première extrémité de la barre d'élément ressort (436) comprend un cliquet (433) qui est agencé pour s'encliqueter de façon irréversible dans une rainure (434) dans la structure de poignée (45) lorsque l'étui protecteur d'aiguille (42) arrive à la deuxième position, établissant ainsi une seconde charnière (437) entre ladite extrémité de la barre (436) et la structure de poignée (45).

5. Dispositif selon l'une quelconque des revendications précédentes, **caractérisé par le fait que** l'élément ressort (43) comprend une barre élastique (436) qui est reliée sur une première extrémité à l'étui protecteur d'aiguille (42) par une première charnière (431) et est reliée sur l'autre extrémité à la structure de poignée (45) par une seconde charnière (437).

6. Dispositif selon l'une quelconque des revendications précédentes, **caractérisé par le fait que** l'étui protecteur d'aiguille (42) comprend un élément (441) qui est monté de façon coulissante (52, 463) sur la structure de poignée (45), de telle sorte que l'étui protecteur, avec l'élément coulissant (441), est déplaçable de façon linéaire (52) entre la première position et une troisième position.

7. Dispositif selon l'une quelconque des revendications précédentes, **caractérisé par** un ou plusieurs mécanismes de verrouillage (46, 46', 44, 425) pour verrouiller l'étui protecteur d'aiguille (42) dans une position dans laquelle l'aiguille (41) se situe dans l'étui protecteur (42).

8. Dispositif selon l'une quelconque des revendications précédentes, **caractérisé par le fait que** l'étui protecteur d'aiguille (42) comprend un élément (441) qui est monté de façon coulissante (52, 463) sur la structure de poignée (45) et est déplaçable de façon linéaire (52) entre la première position et une troisième position, et un mécanisme de verrouillage rotatif (46) est réalisé sous la forme d'une liaison fentelanguette, une fente (461) étant agencée sur l'étui protecteur (42) et la languette (462) étant agencée sur l'élément coulissant (441), ou inversement, et la fente (461) et la languette (462) ne s'accouplant pas lorsque l'élément coulissant (441) est dans une position et s'accouplant lorsque l'élément coulissant est dans l'autre position, bloquant ainsi le mouvement de rotation (51) de l'étui protecteur d'aiguille (42) et établissant un verrouillage de l'étui protecteur d'aiguille.

9. Dispositif selon l'une quelconque des revendications précédentes, **caractérisé par le fait que** l'étui protecteur d'aiguille (42) comprend un élément (441) qui est monté de façon coulissante (52, 463) sur la structure de poignée (45) et est déplaçable de façon linéaire (52) entre la première position et une troisième position, et un mécanisme de verrouillage rotatif (46') comprenant une fente de guidage (463), la charnière (424) de l'étui protecteur d'aiguille étant située à l'extérieur de la fente de guidage (463) lorsque l'élément coulissant (441) est dans une position et étant située à l'intérieur de la fente de guidage lorsque l'élément coulissant est dans l'autre position, bloquant ainsi le mouvement de rotation (51) de l'étui protecteur d'aiguille (42) en rendant la charnière (424) non fonctionnelle; et établissant un verrouillage de l'étui protecteur d'aiguille dans la première position.

10. Dispositif selon l'une quelconque des revendications précédentes, **caractérisé par le fait que** l'étui protecteur d'aiguille (42) comprend un élément (441) qui est monté de façon coulissante (52, 463) sur la structure de poignée (45), de telle sorte que l'étui protecteur d'aiguille, avec l'élément coulissant, est déplaçable de façon linéaire (52) entre la première position et une troisième position, et un mécanisme de verrouillage en translation (44) est réalisé sous la forme d'un mécanisme à cliquet linéaire (441, 442), l'élément coulissant servant d'élément rochet linéaire (441) du mécanisme à cliquet linéaire et la structure de poignée comprenant un cliquet à ressort (442) du mécanisme à cliquet linéaire, ou inversement.

11. Dispositif selon l'une quelconque des revendications précédentes, **caractérisé par le fait que** l'étui protecteur d'aiguille comprend deux fentes parallèles (423) dans les parois latérales (428) de l'étui protecteur (42).

12. Interface de site de perfusion ou de capteur ayant un dispositif d'insertion (4) selon l'une quelconque des revendications 1 à 11, une aiguille d'insertion (41) du dispositif d'insertion (4) étant disposée dans une canule de perfusion (12) de l'interface ou dans un capteur de l'interface.

13. Ensemble de perfusion ayant un dispositif d'insertion (4) selon l'une quelconque des revendications 1 à 11.
